(19) Europäisches Patentamt
European Patent Office
Office européen des brevets

(11) **EP 2 204 178 A1**

(12) **EUROPEAN PATENT APPLICATION**
published in accordance with Art. 153(4) EPC

(43) Date of publication:
**07.07.2010 Bulletin 2010/27**

(21) Application number: **08845396.4**

(22) Date of filing: **28.10.2008**

(51) Int Cl.:
**A61K 33/08** (2006.01)   **A61K 33/30** (2006.01)
**A61P 1/04** (2006.01)   **A61P 1/10** (2006.01)
**A61P 3/02** (2006.01)

(86) International application number:
**PCT/JP2008/069988**

(87) International publication number:
**WO 2009/057796 (07.05.2009 Gazette 2009/19)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **29.10.2007   JP 2007280070
25.04.2008   JP 2008115807**

(71) Applicant: **KYOWA CHEMICAL INDUSTRY CO.,
LTD.
Takamatsu-shi
Kagawa-ken 761-0113 (JP)**

(72) Inventors:
• **KATSUKI, Keiko**
**Sakaide-shi
Kagawa 762-0012 (JP)**
• **OKADA, Akira**
**Sakaide-shi
Kagawa 762-0012 (JP)**
• **KITAJIMA, Hideaki**
**Kita-gun
Kagawa 761-0705 (JP)**

(74) Representative: **Benson, John Everett
J.A. Kemp & Co.
14 South Square
Gray's Inn
GB-London WC1R 5JJ (GB)**

(54) **LAXATIVE AGENT**

(57)   A laxative agent comprising composite magnesium oxide particles represented by the following formula (1) as an effective component:

$$(Mg^{2+})_{1-x}(Zn^{2+})_xO \qquad (1)$$

$$(x \text{ is } 0.0001 \text{ to } 0.3.).$$

The laxative agent is excellent in the effect of protecting the mucosa of a digestive organ.

**Description**

Technical Field

The present invention relates to a laxative agent comprising composite magnesium oxide particles as an effective component. More specifically, it relates to a laxative agent comprising composite magnesium oxide particles prepared by adding a small amount of zinc (Zn) to magnesium oxide particles as an effective component.

Background of the Art

A laxative agent comprising magnesium oxide particles as an effective component is produced and marketed as a tablet, granule or capsule. This laxative agent has a problem that it is difficult to be taken because it must contain a large amount of magnesium oxide particles to obtain satisfactory laxative action. Since a conventional laxative agent stimulates the intestines to cause peristalsis so as to promote laxative action, it causes an abdominal pain and an adverse effect such as damage to the intestinal wall when it is taken for a long time.

To solve the above problems, there is proposed a laxative agent comprising magnesium oxide particles, a binder which can exhibit laxative action and a disintegrant which can exhibit laxative action (patent document 1).

There is further proposed a laxative tablet which has excellent acid reactivity and does not stimulate the intestines directly by limiting the specific surface area of each magnesium oxide particle to a specific range (patent document 2).

In the case of a tablet, when the content of magnesium oxide particles is made high and the tablet is hard, the tablet is hardly disintegrated and the development of laxative action is slowed down. To solve this, a large amount of a disintegrant is blended and therefore the content of the magnesium oxide particles in the tablet is reduced.

There is also proposed a tablet having an increased content of magnesium oxide particles having an average secondary particle diameter measured by a laser diffraction scattering method of 0.5 to 10 $\mu$m in order to enhance its antacid and laxative effect by mixing a small amount of a disintegrant (patent document 3).

(patent document 1) JP-A 9-40561
(patent document 2) JP-A 2001-48792
(patent document 3) JP-A 2003-146889

Disclosure of the Invention

Various studies are now under way to enhance the performance of magnesium oxide particles as a laxative agent. However, attempts are not being made to add an additional effect except the laxative effect to the magnesium oxide particles. Particularly, the influence of the magnesium oxide particles upon the mucosa of a digestive organ is not studied.

It is therefore an object of the present invention to provide a laxative agent comprising magnesium oxide particles as an effective component and having the excellent effect of protecting the mucosa of a digestive organ.

The inventors of the present invention have studied the influence of magnesium oxide particles on the mucosa of a digestive organ. As a result, they have found that, when zinc is contained in the magnesium oxide particles, the obtained product exhibits not only laxative action but also the effect of protecting the inner walls of digestive organs to suppress ulceration. The present invention has been accomplished based on this finding.

That is, according to the present invention, there are provided the following:

1. A laxative agent comprising composite magnesium oxide particles represented by the following formula (1) as an effective component:

$$(Mg^{2+})_{1-x}(Zn^{2+})_x O \qquad\qquad (1)$$
$$(x \text{ is } 0.0001 \text{ to } 0.3.).$$

2. The laxative agent according to the paragraph 1, wherein x in the formula (1) is 0.0005 to 0.2.

3. The laxative agent according to the paragraph 1, wherein the average secondary particle diameter measured by a laser diffraction scattering method of the composite magnesium oxide particles is 0.5 to 25 $\mu$m.

4. The laxative agent according to the paragraph 1, wherein the specific surface area measured by a BET method of the composite magnesium oxide particles is 20 to 100 $m^2$/g.

5. The laxative agent according to the paragraph 1, wherein the specific surface area measured by a BET method of the composite magnesium oxide particles is 20 to 70 $m^2$/g.

6. The laxative agent according to the paragraph 1 which contains the composite magnesium oxide particles in an amount of 88 to 97 wt%.

7. The laxative agent according to the paragraph 1 which is in the form of a tablet.

8. A zinc supplement comprising composite magnesium oxide particles represented by the following formula (1) as an effective component:

$$(Mg^{2+})_{1-x}(Zn^{2+})_xO \qquad\qquad (1)$$

$$(x \text{ is } 0.0001 \text{ to } 0.3.).$$

9. An agent for protecting the mucosa of a digestive organ, which comprises composite magnesium oxide particles represented by the following formula (1) as an effective component:

$$(Mg^{2+})_{1-x}(Zn^{2+})_xO \qquad\qquad (1)$$

$$(x \text{ is } 0.0001 \text{ to } 0.3.).$$

10. The agent according to the paragraph 9 which is an agent for protecting the mucosa of the stomach.

11. Use of composite magnesium oxide particles represented by the following formula (1) for the manufacture of a laxative agent:

$$(Mg^{2+})_{1-x}(Zn^{2+})_xO \qquad\qquad (1)$$

$$(x \text{ is } 0.0001 \text{ to } 0.3.).$$

12. Use of composite magnesium oxide particles represented by the following formula (1) for the manufacture of an agent for protecting the mucosa of a digestive organ:

$$(Mg^{2+})_{1-x}(Zn^{2+})_xO \qquad\qquad (1)$$

$$(x \text{ is } 0.0001 \text{ to } 0.3.).$$

13. Composite magnesium oxide particles represented by the following formula (1) for a laxative treatment:

$$(Mg^{2+})_{1-x}(Zn^{2+})_xO \qquad\qquad (1)$$

$$(x \text{ is } 0.0001 \text{ to } 0.3.).$$

14. Composite magnesium oxide particles represented by the following formula (1) for the treatment of gastric ulceration:

$$(Mg^{2+})_{1-x}(Zn^{2+})_xO \qquad\qquad (1)$$

$$(x \text{ is } 0.0001 \text{ to } 0.3.).$$

Best Mode for Carrying Out the Invention

<composite magnesium oxide particles>

The composite magnesium oxide particles are represented by the following formula (1).

$$(\mathrm{Mg}^{2+})_{1-x}(\mathrm{Zn}^{2+})_x\mathrm{O} \qquad\qquad (1)$$

In the above formula, x is 0.0001 to 0.3, preferably 0.0005 to 0.3, more preferably 0.0005 to 0.2. When x is larger than 0.3, the total amount of essential minerals including Zn ingested from food may exceed the required amount. When x is smaller than 0.0001, the particles tend not to be able to exhibit the effect of protecting the mucosa of a digestive organ.

The composite magnesium oxide particles which contain zinc (Zn) in a small amount as a solid solution are obtained. This is a compound having the same crystal structure as that of magnesium oxide particles. The composite magnesium oxide particles are not a mixture of magnesium oxide particles and zinc but have a structure that zinc atoms enter in the crystal structure of magnesium oxide. The present invention is characterized by the finding that an excellent mucosa protection effect is obtained with a small amount of zinc having a mucosa protection function by introducing the zinc into the crystal structure of magnesium oxide and not simply by mixing it.

The composite magnesium oxide particles show the same diffraction pattern as that of magnesium oxide particles according to a powder X-ray diffraction method. When a normal amount of the composite magnesium oxide particles is taken as a laxative agent, as the amount of solid-dissolved zinc is smaller than the required amount of zinc as an essential mineral required for the human being, it is safe and zinc can be supplemented. Since the composite magnesium oxide particles are a solid solution with Zn, it is easily absorbed the intestines and does not damage the intestinal wall.

The average secondary particle diameter measured by a laser diffraction scattering method of the composite magnesium oxide is preferably 0.5 to 25 $\mu$m, more preferably 5 to 20 $\mu$m.

The specific surface area measured by a BET method of the composite magnesium oxide is preferably 20 to 100 $\mathrm{m}^2$/g, more preferably 20 to 70 $\mathrm{m}^2$/g. When the specific surface area falls within this range, the composite magnesium oxide shows excellent acid reactivity and can be easily tableted.

The composite magnesium oxide particles may be in any form such as powder, granule, tablet, capsule or slurry.

<method of manufacturing composite magnesium oxide particles>

The composite magnesium oxide particles are manufactured by adding an alkaline substance to an aqueous solution containing a magnesium ion and zinc ion in an amount of almost the same equivalent as the total equivalent of these cations, reacting them under agitation and optionally further hydrothermally treating the reaction product in an autoclave at 100 to 200˚C. Thereafter, the reaction product is washed with water, dehydrated and dried. After it is calcined, commonly used means such as grinding and classification are suitably employed to prepare the composite magnesium oxide particles. Calcination is preferably carried out at 300 to 1,200˚C, more preferably 400 to 900˚C for 0.1 to 10 hours. Magnesium nitrate and magnesium chloride are preferably used as a source material for the magnesium ion. Zinc nitrate and zinc chloride are preferably used as a source material for the zinc ion. Sodium hydroxide is preferred as the alkaline substance.

The obtained powders may be taken directly as a laxative agent or may be granulated or tableted to be taken.

<granule>

The granule is prepared by mixing together a binder, a disintegrant and composite magnesium oxide particles and dry granulating the resulting mixture. In this case, (1) 88 to 97 wt% of the composite magnesium oxide particles, (2) 1 to 10 wt% (preferably 1 to 8 wt%) of a binder and (3) 1 to 10 wt% (preferably 1 to 5 wt%) of a disintegrant are mixed together by means of a container type, V type or W type mixer and the resulting mixture is granulated to obtain granular particles. Granulation is preferably carried out at a low pressure by using a dry granulator. The roll pressure in this case is preferably 3 to 12 MPa, more preferably 4 to 8 MPa. The granulated sheet-like product is ground by an oscillator type grinder to obtain granular particles. The screen to be set in the oscillator has a mesh size of preferably 0.7 to 1.2 mm, more preferably 0.8 to 1.0 mm. Granular particles having an average particle diameter of 0.25 to 2.00 mm and an apparent density of 0.5 to 0.7 g/ml are thus obtained.

The tablet may contain a vehicle, a binder, a disintegrant and a lubricant as required, in addition to the composite magnesium oxide particles. The content of the composite magnesium oxide particles in the tablet is preferably 88 to 97 wt%, more preferably 88 to 96 wt%, much more preferably 90 to 95 wt%. The composite magnesium oxide to be used for tableting may be particulate, powdery or granular.

Examples of the binder include carboxymethyl cellulose sodium and low-substituted hydroxypropyl cellulose. The content of the binder in the tablet is preferably 1 to 10 wt%, more preferably 1 to 8 wt%.

Examples of the vehicle include crystalline cellulose and starch (such as corn starch). The content of the vehicle in the tablet is preferably 1 to 10 wt%, more preferably 1 to 8 wt%.

Examples of the disintegrant include starch (such as corn starch), carboxymethyl cellulose calcium, carmellose, low-substituted hydroxypropyl cellulose, crosscarmellose sodium, carmellose calcium and carboxy starch sodium. These disintegrants may be used in combination of two or more. Crosscarmellose sodium and carboxy starch sodium are particularly preferred as the disintegrant. Since these disintegrants make disintegrate the tablet with a much smaller amount than a conventional disintegrant, the content of the disintegrant can be reduced. A tablet which has excellent stability and rarely changes of aging can be obtained. The most preferred disintegrant is crosscarmellose sodium. The content of the disintegrant in the tablet is preferably 1 to 10 wt%, more preferably 1 to 5 wt%.

The tablet may be prepared by mixing a binder, a disintegrant, a vehicle and a lubricant with the composite magnesium oxide particles and tableting by the direct compression system.

0.2 to 2 wt% of the lubricant may be added to the above granule to prepare the tablet. Examples of the used lubricant include stearic acid and its salts (Na, Mg and Ca salts) thereof. Stearates, particularly calcium stearate and magnesium stearate are preferred. Calcium stearate is the most effective. When the amount of the lubricant is too large, disintegration is retarded and when the amount is too small, the lubricant adheres to a mortar and a pestle. Therefore, the amount of the lubricant is preferably 0.2 to 2 wt%, more preferably 0.8 to 1.2 wt%.

The average particle diameter of the granules in this case is preferably 0.25 to 0.5 mm. At least one of the above binders is contained in an amount of 1 to 10 wt%, preferably 1 to 5 wt% based on the tablet. At least one of the above disintegrants is contained in an amount of 5 to 20 wt%, preferably 5 to 10 wt% based on the tablet.

When hard composite magnesium oxide particles are used, the disintegration time of the tablet becomes long and the development of a laxative effect becomes slow. Therefore, it is desired to obtain a tablet having a short disintegration time by specifying composite magnesium oxide particles and a disintegrant, and the pressure of dry granulation for the molding of a granule is preferably 4 to 8 MPa.

The present invention includes a method of using the composite magnesium oxide particles represented by the following formula (1) as a laxative agent:

$$(Mg^{2+})_{1-x}(Zn^{2+})_xO \qquad\qquad (1)$$

$$(x \text{ is } 0.0001 \text{ to } 0.3.).$$

The composite magnesium oxide particles of the present invention may also be used as a zinc supplement. Therefore, the present invention includes a zinc supplement comprising the composite magnesium oxide particles represented by the following formula (1) as an effective component:

$$(Mg^{2+})_{1-x}(Zn^{2+})_xO \qquad\qquad (1)$$

$$(x \text{ is } 0.0001 \text{ to } 0.3.).$$

The present invention also includes a method of using the composite magnesium oxide particles represented by the formula (1) as a zinc supplement.

The composite magnesium oxide particles of the present invention may also be used as an agent for protecting the mucosa of a digestive organ. Therefore, the present invention includes an agent for protecting the mucosa of a digestive organ, comprising the composite magnesium oxide particles represented by the following formula (1) as an effective component:

$$(Mg^{2+})_{1-x}(Zn^{2+})_x O \qquad\qquad (1)$$

$$(x \text{ is } 0.0001 \text{ to } 0.3.).$$

Particularly, it can be used as an agent for protecting the mucosa of the stomach. The present invention includes a method of using the composite magnesium oxide particles represented by the formula (1) as an agent for protecting the mucosa of a digestive organ.

The present invention includes use of the composite magnesium oxide particles represented by the formula (1) for the manufacture of a laxative agent. The present invention includes use of the composite magnesium oxide particles represented by the formula (1) for the manufacture of an agent for protecting the mucosa of a digestive organ.

The present invention includes the composite magnesium oxide particles represented by the formula (1) for a laxative treatment. The present invention also includes the composite magnesium oxide particles represented by the formula (1) for the treatment of gastric ulceration.

Examples

The following examples are provided to further illustrate the present invention. In the examples, (a) average secondary particle diameter, (b) BET specific surface area, (c) zinc (Zn) analysis, (d) tablet hardness, (e) disintegration test and (f) abrasion of the composite magnesium oxide particles were measured by the following methods.

(a) Average secondary particle diameter of composite magnesium oxide particles

This was measured by using the MICROTRAC particle size distribution meter SPA type (of LEEDS & NORTHRUP).

700 mg of a sample powder was added to 70 ml of water and dispersed in the water for 3 minutes with ultrasonic waves (Model US-300 of NISSEI Co., Ltd., current of 300 $\mu$A), 2 - 4 ml of the obtained dispersion was collected and put into the sample chamber of the above particle size distribution meter containing 250 ml of deaerated water, the meter was activated to circulate the suspension for 8 minutes, and then the particle size distribution of the sample was measured. The above measurement was made twice in total, and the arithmetic average value of the 50 % accumulative secondary particle diameters obtained from the above measurements was calculated and taken as the average secondary particle diameter of the sample.

(b) BET specific surface area of composite magnesium oxide particles

This was measured by a liquid nitrogen adsorption method.

(c) Analysis of zinc (Zn)

This was measured by atomic absorption method.

(d) Tablet hardness

The tablet hardness was measured by using the 8M tablet hardness meter of Dr. Schleuniger Pharmatron. The average value and standard deviation of 10 tablets were obtained.

(e) Disintegration test

This was conducted in accordance with the general test method of the Japanese Pharmacopoeia Fifteenth Edition using water as a test liquid.

(f) Abrasion

This was based on the reference information of the Japanese Pharmacopoeia Fifteenth Edition Supplement I.

Example 1

An aqueous solution of a mixture of magnesium nitrate and zinc nitrate (magnesium nitrate concentration of 1.30 mol/L, zinc nitrate concentration of $1.3 \times 10^{-4}$ mol/L, designated as solution A) and a 6.5 N solution of sodium hydroxide (designated as solution B) were continuously injected into a reactor containing water under agitation by using a metering pump. A reaction was carried out at 40˚C and a pH of 10.5 and the retention time of the reaction solution was 30 minutes, and a reaction suspension overflown from the reactor was taken out for 4 hours. After the reaction solution was separated by filtrated, washed with water and dried at 110˚C for 24 hours, the obtained product was ground and sieved to obtain composite magnesium hydroxide particles.

The composite magnesium hydroxide particles were then calcined in a calcining furnace at 700˚C for 2 hours to obtain composite magnesium oxide particles having the following composition.

Composition: $Mg_{0.9999}Zn_{0.0001}O$

Example 2

An aqueous solution of a mixture of a magnesium chloride reagent and a zinc chloride reagent (magnesium chloride concentration of 1.30 mol/L, zinc chloride concentration of $7.8 \times 10^{-4}$ mol/L, designated as solution A) and a 6.5 N solution of sodium hydroxide (designated as solution B) were reacted with one another in the same manner as in Example 1 to obtain composite magnesium hydroxide particles. The composite magnesium hydroxide particles were then baked in a firing furnace at 750˚C for 2 hours to obtain composite magnesium oxide particles having the following composition. Composition: $Mg_{0.9994}Zn_{0.0006}O$

Example 3

An aqueous solution of a mixture of a magnesium nitrate reagent and a zinc nitrate reagent (magnesium nitrate concentration of 1.30 mol/L, zinc nitrate concentration of $5.3 \times 10^{-3}$ mol/L, designated as solution A) and a 6.5 N aqueous solution of sodium hydroxide (designated as solution B) were reacted with one another in the same manner as in Example 1, and 700 ml of a reaction suspension overflown from a reactor was reacted at 80˚C for 2 hours. After the reaction suspension was cooled, filtrated, washed with water and dried at 110˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition.

Composition: $Mg_{0.996}Zn_{0.004}O$

Example 4

An aqueous solution of a mixture of a magnesium nitrate reagent and a zinc nitrate reagent (magnesium nitrate concentration of 1.50 mol/L, zinc nitrate concentration of $9.1 \times 10^{-3}$ mol/L, designated as solution A) and a 6.5 N aqueous solution of sodium hydroxide (designated as solution B) were reacted with one another in the same manner as in Example 1, and 700 ml of a reaction suspension overflown from a reactor was transferred into an autoclave to be hydrothermally reacted at 110˚C for 6 hours. After the reaction suspension was cooled, filtrated, washed with water and dried at 110˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition. Composition: $Mg_{0.994}Zn_{0.006}O$

Example 5

An aqueous solution of a mixture of a magnesium nitrate reagent and a zinc nitrate reagent (magnesium nitrate concentration of 2.02 mol/L, zinc nitrate concentration of $4.04 \times 10^{-2}$ mol/L, designated as solution A) and a 3.4 N aqueous solution of sodium hydroxide (designated as solution B) were reacted with one another in the same manner as in Example 1, and 650 ml of a reaction suspension overflown from a reactor was transferred into an autoclave to be hydrothermally reacted at 170˚C for 3 hours. After the reaction suspension was cooled, filtrated, washed with water and dried at 105˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition. Composition: $Mg_{0.990}Zn_{0.010}O$

Example 6

An aqueous solution of a mixture of a magnesium nitrate reagent and a zinc nitrate reagent (magnesium nitrate concentration of 1.30 mol/L, zinc nitrate concentration of $6.84 \times 10^{-2}$ mol/L, designated as solution A) and a 6.5 N aqueous solution of sodium hydroxide (designated as solution B) were reacted with one another in the same manner as in Example 1, and 700 ml of a reaction suspension overflown from a reactor was transferred into an autoclave to be hydrothermally reacted at 100˚C for 3 hours. After the reaction suspension was cooled, filtrated, washed with water and dried at 110˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition. Composition: $Mg_{0.95}Zn_{0.05}O$

Example 7

An aqueous solution of a mixture of a magnesium nitrate reagent and a zinc nitrate reagent (magnesium nitrate concentration of 1.30 mol/L, zinc nitrate concentration of 0.144 mol/L, designated as solution A) and a 6.5 N aqueous solution of sodium hydroxide (designated as solution B) were reacted with one another in the same manner as in Example 1, and 700 ml of a reaction suspension overflown from a reactor was transferred into an autoclave to be hydrothermally reacted at 100˚C for 3 hours. After the reaction suspension was cooled, filtrated, washed with water and dried at 110˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition. Composition: $Mg_{0.90}Zn_{0.10}O$

Example 8

An aqueous solution of a mixture of a magnesium nitrate reagent and a zinc nitrate reagent (magnesium nitrate concentration of 1.30 mol/L, zinc nitrate concentration of 0.325 mol/L, designated as solution A) and a 6.5 N aqueous solution of sodium hydroxide (designated as solution B) were reacted with one another in the same manner as in Example 1, and 700 ml of a reaction suspension overflown from a reactor was transferred into an autoclave to be hydrothermally reacted at 100˚C for 3 hours. After the reaction suspension was cooled, filtrated, washed with water and dried at 110˚C for 24 hours, the obtained product was ground and sieved to obtain composite magnesium hydroxide particles. The composite magnesium hydroxide particles were then calcined in a calcining furnace at 700˚C for 2 hours to obtain composite magnesium oxide particles having the following composition. Composition: $Mg_{0.80}Zn_{0.20}O$

The characteristic properties of the composite magnesium oxide particles obtained in Examples 1 to 8 are shown in Table 1 below.

Table 1

| Example | | 1 | 2 | 3 | 4 | 5 | 6 | 7 | 8 |
|---|---|---|---|---|---|---|---|---|---|
| Zn | (wt%) | 0.02 | 0.10 | 0.65 | 0.97 | 1.59 | 7.72 | 14.72 | 26.95 |
| Average secondary particle diameter | ($\mu$m) | 10.9 | 10.7 | 8.0 | 16.0 | 0.52 | 15.5 | 18.0 | 17.0 |
| BET specific surface area | ($m^2$/g) | 59 | 41 | 26 | 51 | 57 | 48 | 39 | 56 |

Example 9: tablet

17.94 Kg of the composite magnesium oxide particles obtained in the same manner as in Example 3, 1.24 Kg of crystalline cellulose and 0. 62 Kg of cross carmellose sodium were mixed together by a container type mixer, and the resulting mixture was granulated by a roll molding granulator to produce granules. 19 Kg of the granules having a granule diameter of 0.3 to 0.4 mm and 0.19 Kg of calcium stearate were mixed together by a container type mixer to obtain granules which were then tableted by a rotary tableting machine having 24 pestles of 17.5R having a diameter of 10.5 mm at a tableting pressure of 15 KN to obtain composite magnesium oxide tablets, each having a weight of 580 mg and a thickness of 5.1 mm. The amounts of the above substances and the hardness, disintegration time and abrasion of the tablet are shown in Table 6.

Example 10: tablet

19.78 Kg of the composite magnesium oxide particles obtained in Example 3, 0.56 Kg of crystalline cellulose, 0.36 Kg of corn starch and 0.64 Kg of crosscarmellose sodium were mixed together by a container type mixer, and the resulting mixture was granulated by a roll molding granulator to produce granules. 19 Kg of the granules having a granule diameter of 0.3 to 0.4 mm and 0.21 Kg of calcium stearate were mixed together by a container type mixer to obtain granules which were then tableted by a rotary tableting machine having 24 pestles of 12R having a diameter of 8 mm at a tableting pressure of 8 KN to obtain composite magnesium oxide tablets, each having a weight of 285 mg and a thickness of 4.4 mm. The amounts of the above substances and the hardness, disintegration time and abrasion of the tablet are shown in Table 6.

Table 6

| Composition | Tablet (Example 9) | Tablet (Example 10) |
|---|---|---|
| Composite magnesium oxide mg (wt%) | 520(89.7) | 260(91.2) |
| Crystalline cellulose mg (wt%) | 36(6.2) | 8(2.8) |
| Corn starch mg (wt%) | - | 5(1.8) |
| Crosscarmellose sodium mg (wt%) | 18(3.1) | 9(3.2) |
| Calcium stearate mg (wt%) | 6(1.0) | 3(1.1) |
| Total mg (wt%) | 580(100) | 285(100.1) |
| Tablet hardness (KV) | 110-138 | 71-86 |
| Disintegration time (second) | 8-11 | 8-9 |
| Abrasion (%) | 0.9-1.21 | 0.3-0.51 |

Example 11: gastric mucosa damage test

A water immersion stress gastric mucosa damage test was conducted on male rats (SPF) using the composite magnesium oxide particles obtained in Example 5. For comparison, magnesium oxide manufactured by Kyowa Chemical Industry Co., Ltd. was used.

(i) test method

test group constitution

| | | |
|---|---|---|
| Control group (media) | | 6 rats |
| Composite magnesium oxide particles (present invention) | 100mg/Kg | 6 rats |
| Magnesium oxide particles (comparison) | 100mg/Kg | 6 rats |

(ii) administration method

administration route: oral administration
applied dose: 5 mL/Kg
administration means: administered by using a disposable injection cylinder and a sonde for oral administration
administration time: administered 60 minutes before the preparation of a gastric mucosa damaged model

After about 24 hours of fasting, the rats were put into a stress cage (manufactured by Natsume Seisakusho Co., Ltd.), and the cage was immersed in a water tank at 23°C. 6 hours after that, the blood was removed from the rats to kill them under anesthesia with pentobarbital sodium (40 mg/Kg, i.p.), and the stomachs of these rats were extracted. 10 mL of a 1 % formalin solution was injected into the stomachs to immerse them in the solution for 10 minutes or more. The stomachs were cut open along the greater curvature to measure the length (mm) of a damage by a stereomicroscope. The total of damages of each rat was taken as the damage coefficient of the rat. The results are shown in Table 2. The results show that the composite magnesium oxide particles of the present invention are more effective.

Table 2

| Type of agent | Applied dose (mg/Kg, p.o.) | Number rats | Damage coefficient Inhibition (Lesions)(mm) | Inhibition ratio (%) |
|---|---|---|---|---|
| Reference example [a)] | - | 6 | 41.0±0.2 | - |
| Example 5 (composite magnesium oxide particles) | 300 | 6 | 10.8±2.2*** | 74 |
| Comparative example (magnesium oxide particles) | 300 | 6 | 18.1±3.6*** | 56 |
| a) :0.5%MC(MC = methyl cellulose), 5mL/Kg *** P<0.001 VS Control by Student's T-test | | | | |

Example 12: gastric mucosa damage test

An ethanol-inducted gastric mucosa damage test was conducted on male rats (SPF) by using the composite magnesium oxide particles obtained in Example 5. For comparison, magnesium oxide manufactured by Kyowa Chemical Industry Co., Ltd. was used.

After about 24 hours of fasting, 1 mL of ethanol (99.5%) was orally administered to each rat. The test was conducted in the same manner as in Example 11 except that, 1 hour after the administration of ethanol, the blood was removed from the rats to kill them under anesthesia with pentobarbital sodium (40 mg/Kg, i.p.), and the stomachs of these rats were extracted. The results are shown in Table 3. The results show that the composite magnesium oxide particles of the present invention are effective.

Table 3

| Type of agent | Applied dose (mg/Kg,p.o.) | Number of rats | Damage coefficient (Lesions)(mm) | Inhibition ratio (%) |
|---|---|---|---|---|
| Reference example [a)] | - | 6 | 49.3±6.8 | - |
| Example 5 (composite magnesium oxide particles) | 300 | 6 | 21.2±8.7* | 57 |
| Comparative example (magnesium oxide particles) | 300 | 6 | 25.5±8.9 | 48 |
| a) :0.5%MC(MC = methyl cellulose), 5mL/Kg * P<0.05 VS Control by Student's T-test | | | | |

Example 13: gastric mucosa damage test

An indometacin-inducted gastric mucosa damage test was conducted on male rats (SPF) by using the composite magnesium oxide particles obtained in Example 5. For comparison, magnesium oxide manufactured by Kyowa Chemical Industry Co., Ltd. was used.

After about 24 hours of fasting, 30 mg/Kg of indometacin (15 mg/mL: suspended by using a 0. 5 % methylcellulose aqueous solution) was subcutaneously administered to each rat. The test was conducted in the same manner as in Example 11 except that, 5 hours after the administration of indometacin, the blood was removed from the rats to kill them under anesthesia with pentobarbital sodium (40 mg/Kg, i.p.), and the stomachs of these rats were extracted. The results are shown in Table 4. The results show that the composite magnesium oxide particles of the present invention and the magnesium oxide particles of the comparative sample are both effective.

Table 4

| Type of agent | Applied dose (mg/Kg, p.o.) | Number of rats | Damage coefficient (Lesions)(mm) | Inhibition ratio (%) |
|---|---|---|---|---|
| Reference example [a] | - | 6 | $13.8 \pm 2.4$ | - |
| Example 5 (composite magnesium oxide particles) | 300 | 6 | $0.8 \pm 0.6$** | 94 |
| Comparative example (magnesium oxide particles) | 300 | 6 | $0.0 \pm 0.0$** | 100 |
| a) :0.5%MC(MC = methyl cellulose), 5mL/Kg ** $P<0.01$ VS Control by Student's T-test | | | | |

Example 14: gastric mucosa damage test

An aspirin-inducted gastric mucosa damage test was conducted on male rats (SPF) by using the composite magnesium oxide particles obtained in Example 5. For comparison, magnesium oxide manufactured by Kyowa Chemical Industry Co., Ltd. was used.

After about 24 hours of fasting, 125 mg/Kg of aspirin

(62.5 mg/mL: suspended by using a methylcellulose aqueous solution) was orally administered to each rat twice every 2 hours. The test was conducted in the same manner as in Example 11 except that, 4 hours after the second administration of aspirin, the blood was removed from the rats to kill them under anesthesia with pentobarbital sodium (40 mg/Kg, i.p.), and the stomachs of these rats were extracted. The results are shown in Table 5. The results show that the composite magnesium oxide particles of the present invention are effective.

Table 5

| Type of agent | Applied dose (mg/Kg, p.o.) | Number of rats | Damage coefficient (Lesions)(mm) | Inhibition ratio (%) |
|---|---|---|---|---|
| Reference example [a] | - | 6 | $48.8 \pm 8.7$ | - |
| Example 5 (composite magnesium oxide particles) | 300 | 6 | $22.7 \pm 3.2$* | 53 |
| Comparative example (magnesium oxide particles) | 300 | 6 | $27.8 \pm 4.3$ | 43 |
| a) :0.5%MC(MC = methyl cellulose), 5mL/Kg * $P<0.05$ VS Control by Aspirin-Welch's T-test | | | | |

Example 15: gastric ulceration test

The influence of the composite magnesium oxide particles obtained in Example 3 upon gastric ulceration was investigated using male rats (SPF). For comparison, magnesium oxide particles (MgO) manufactured by Kyowa Chemical Industry Co., Ltd. were used.

(test method)

test group constitution

control group (media)                                      6 rats
composite magnesium oxide particles      100mg/Kg      6 rats

(continued)

| magnesium oxide particles | 100mg/Kg | 6 rats |

The rats were abdominally operated under anesthesia with pentobarbital sodium (10 mg/Kg, i.p.), 30 $\mu$L of 20 % acetic acid was injected into the submucosal coat at the boundary between the body of stomach and the vestibular region of the pyloric from the serosal side to prepare acetic acid ulceration models. Three days after the preparation of the ulceration models, they were divided into groups, and a test substance was orally administered to these models in an amount of 100 mg/Kg once each day for 10 days repeatedly. On the day following the last administration, the stomachs of the models were extracted under anesthesia with pentobarbital sodium (40 mg/Kg, i.p.) to measure the long diameter and short diameter (mm) of an ulcer. The product (mm$^2$) of the long diameter and the short diameter was taken as a damage coefficient, and the average value$\pm$standard deviation of 6 rats is shown. The results are shown in Table 7. The results show that the composite magnesium oxide particles of the present invention are also effective for the treatment of gastric ulceration.

Table 7

| Type of agent | Applied dose (mg/Kg, p.o.) | Number of rats | Damage coefficient (Lesions area) (mm$^2$) | Inhibition ratio of gastric lesion(%) |
|---|---|---|---|---|
| Reference example [a) | - | 6 | 6.4$\pm$0.7 | - |
| Example 3 (composite magnesium oxide particles) | 300 | 6 | 4.7$\pm$1.1 | 27 |
| Comparative example (magnesium oxide particles) | 300 | 6 | 5.2$\pm$1.0 | 19 |
| a) 0.5% methylcellulose (5mL/Kg) | | | | |

Example 16: laxative action test

Five healthy volunteers took the tablets obtained in Example 10 three times a day for 5 days with water; 2 tablets after breakfast, 3 tablets after lunch and 3 tablets after supper. Then their defecations were observed. The results are shown in Table 8 below. In the table, "first day" means the day following the day when they took the tablets. In the case of a commercially available laxative agent which stimulates peristalsis of the intestines, it caused a stomachache whereas the laxative agent of the present invention did not cause any stomachache because it does not vibrate the intestines.

Table 8

| Subject | First day | Second day | Third day | Fourth day | Fifth day | Other symptom |
|---|---|---|---|---|---|---|
| A | ○ | ○ | ○ | ○ | × | none |
| B | ○ | ○ | ○ | ○ | ○ | none |
| C | △ | ○ | ○ | △ | ○ | none |
| D | ○ | ○ | ○ | ○ | ○ | none |
| E | ○ | ○ | ○ | ○ | ○ | none |
| ○: Loose passage × : diarrhea △ : normal | | | | | | |

Effect of the Invention

The laxative agent of the present invention has excellent antacid and laxative action. The laxative agent of the present invention is excellent in the effect of protecting the mucosa of the inner wall of digestive organs such as esophagus, stomach, duodenum, small intestine or large intestine and can inhibit ulceration. According to the laxative agent of the present invention, zinc which tends to be insufficient for the human body can be supplied.

Industrial Applicability

The laxative agent of the present invention is useful as an antacid and laxative agent. The laxative agent of the present invention is also useful as a zinc supplement.

**Claims**

1.  A laxative agent comprising composite magnesium oxide particles represented by the following formula (1) as an effective component:

$$(Mg^{2+})_{1-x}(Zn^{2+})_{x}O \qquad\qquad (1)$$
$$(x\ is\ 0.0001\ to\ 0.3.).$$

2.  The laxative agent according to claim 1, wherein x in the formula (1) is 0.0005 to 0.2.

3.  The laxative agent according to claim 1, wherein the average secondary particle diameter measured by a laser diffraction scattering method of the composite magnesium oxide particles is 0.5 to 25 $\mu$m.

4.  The laxative agent according to claim 1, wherein the specific surface area measured by a BET method of the composite magnesium oxide particles is 20 to 100 $m^2$/g.

5.  The laxative agent according to claim 1, wherein the specific surface area measured by a BET method of the composite magnesium oxide particles is 20 to 70 $m^2$/g.

6.  The laxative agent according to claim 1 which contains the composite magnesium oxide particles in an amount of 88 to 97 wt%.

7.  The laxative agent according to claim 1 which is the form of a tablet.

8.  A zinc supplement comprising composite magnesium oxide particles represented by the following formula (1) as an effective component:

$$(Mg^{2+})_{1-x}(Zn^{2+})_{x}O \qquad\qquad (1)$$
$$(x\ is\ 0.0001\ to\ 0.3.).$$

9.  An agent for protecting the mucosa of a digestive organ, which comprises composite magnesium oxide particles represented by the following formula (1) as an effective component:

$$(Mg^{2+})_{1-x}(Zn^{2+})_{x}O \qquad\qquad (1)$$
$$(x\ is\ 0.0001\ to\ 0.3.).$$

10. The agent according to claim 9 which is an agent for protecting the mucosa of the stomach.

11. Use of composite magnesium oxide particles represented by the following formula (1) for the manufacture of a laxative agent:

$$(Mg^{2+})_{1-x}(Zn^{2+})_x O \qquad\qquad (1)$$

$$(x \text{ is } 0.0001 \text{ to } 0.3.).$$

12. Use of composite magnesium oxide particles represented by the following formula (1) for the manufacture of an agent for protecting the mucosa of a digestive organ:

$$(Mg^{2+})_{1-x}(Zn^{2+})_x O \qquad\qquad (1)$$

$$(x \text{ is } 0.0001 \text{ to } 0.3.).$$

13. Composite magnesium oxide particles represented by the following formula (1) for a laxative treatment:

$$(Mg^{2+})_{1-x}(Zn^{2+})_x O \qquad\qquad (1)$$

$$(x \text{ is } 0.0001 \text{ to } 0.3.).$$

14. Composite magnesium oxide particles represented by the following formula (1) for the treatment of gastric ulceration:

$$(Mg^{2+})_{1-x}(Zn^{2+})_x O \qquad\qquad (1)$$

$$(x \text{ is } 0.0001 \text{ to } 0.3.).$$

| INTERNATIONAL SEARCH REPORT | International application No. |
|---|---|
| | PCT/JP2008/069988 |

**A. CLASSIFICATION OF SUBJECT MATTER**
*A61K33/08*(2006.01)i, *A61K33/30*(2006.01)i, *A61P1/04*(2006.01)i, *A61P1/10* (2006.01)i, *A61P3/02*(2006.01)i

According to International Patent Classification (IPC) or to both national classification and IPC

**B. FIELDS SEARCHED**

Minimum documentation searched (classification system followed by classification symbols)
A61K33/08, A61K33/30, A61P1/04, A61P1/10, A61P3/02

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho   1971-2009   Toroku Jitsuyo Shinan Koho   1994-2009

Electronic data base consulted during the international search (name of data base and, where practicable, search terms used)
BIOSIS(STN), CAplus(STN), EMBASE(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 06-065011 A (KAISUI KAGAKU KENKYUJO KABUSHIKI KAISHA), 08 March, 1994 (08.03.94), Full text<br>& EP 544502 A1        & JP 06-072816 A<br>& US 5344636 A        & EP 544502 B1<br>& JP 2911282 B2        & JP 2987262 B2 | 13,14<br>1-14 |
| X<br>Y | JP 08-048606 A (KAISUI KAGAKU KENKYUSHO KABUSHIKI KAISHA), 20 February, 1996 (20.02.96), Full text (Family: none) | 13,14<br>1-14 |

[X] Further documents are listed in the continuation of Box C.          [ ] See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 January, 2009 (09.01.09) | 27 January, 2009 (27.01.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

<div align="center">

**INTERNATIONAL SEARCH REPORT**

</div>

| | International application No. |
|---|---|
| | *PCT/JP2008/069988* |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 11-180808 A  (KYOWA CHEM IND CO., LTD.),<br>06 July, 1999 (06.07.99),<br>Full text<br>& WO 99/30564 A1       & EP 970612 A1<br>& US 6297193 B1       & EP 970612 B1 | 13,14<br>1-14 |
| Y<br>A | JP 2003-146889 A  (KYOWA CHEM IND CO., LTD.),<br>21 May, 2003 (21.05.03),<br>Full text<br>& WO 2003/018034 A1    & US 2004/022872 A1<br>& EP 1421944 A1       & US 7147868 B2<br>& JP 4015485 B2       & EP 1421944 B1 | 1-7,11,13<br>8-10,12,14 |
| Y<br>A | JP 2001-048792 A  (FUJIX INC.),<br>20 February, 2001 (20.02.01),<br>Full text<br>& EP 1004311 A1       & US 6436447 B1<br>& EP 1004311 B1 | 1-7,11,13<br>8-10,12,14 |
| Y<br>A | JP 09-040561 A  (FUJIX KABUSHIKI KAISHA),<br>10 February, 1997 (10.02.97),<br>Full text<br>(Family: none) | 1-7,11,13<br>8-10,12,14 |
| Y<br>A | WONG, S.H. et al, Protection by zinc sulphate<br>against ethanol-induced ulceration: preservation<br>of the gastric mucosal barrier, Pharmacology,<br>1986, Vol.33, No.2, p.94-102, full text,<br>particularly, Abstract | 1-7,9-14<br>8 |
| Y<br>A | JOSEPH, R.M. et al, Protective effects of zinc<br>in indomethacin-induced gastric mucosal injury:<br>evidence for a dual mechanism involving lipid<br>peroxidation and nitric oxide, Aliment Pharmacol<br>Ther, 1999, Vol.13, No.2, p.203-8, full text,<br>particularly, Abstract | 1-7,9-14<br>8 |
| Y<br>A | JP 2001-151681 A  (LINTEC CORP.),<br>05 June, 2001 (05.06.01),<br>Full text<br>& US 2002/001628 A1    & US 6461646 B2 | 1-7,9-14<br>8 |
| Y<br>A | JP 2006-232833 A  (PM INT AG., JP),<br>07 September, 2006 (07.09.06),<br>Full text<br>& US 2006/188608 A1    & EP 1698344 A1<br>& EP 1719519 A1       & EP 1719519 B1<br>& EP 1698344 B1 | 8<br>1-7,9-14 |
| P,X<br>P,A | JP 2008-120703 A  (KAISUI KAGAKU KENKYUJO<br>KABUSHIKI KAISHA),<br>29 May, 2008 (29.05.08),<br>Full text<br>(Family: none) | 9,10,12-14<br>1-8,11 |

Form PCT/ISA/210 (continuation of second sheet) (April 2007)

**EP 2 204 178 A1**

| INTERNATIONAL SEARCH REPORT | International application No. |
|---|---|
| | PCT/JP2008/069988 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   See extra sheet.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable,
the                              payment of a protest fee.

   ☐ The additional search fees were accompanied by the applicant's protest but the applicable protest fee was not paid within the time limit specified in the invitation.

   ☒ No protest accompanied the payment of additional search fees.

Form PCT/ISA/210 (continuation of first sheet (2)) (April 2007)

17

| **INTERNATIONAL SEARCH REPORT** | International application No. |
|---|---|
| | PCT/JP2008/069988 |

Continuation of Box No.III of continuation of first sheet(2)

It is considered that the common matter among the invention of claim 1 "a laxative agent comprising a composite magnesium oxide particle represented by the formula (1) as an active ingredient", the invention of claim 8 "a zinc-supplying agent comprising a composite magnesium oxide particle represented by the formula (1) as an active ingredient", the invention of claim 9 "an agent for protecting a mucous membrane in a digestive organ, which comprises a composite magnesium oxide particle represented by the formula (1) as an active ingredient" and the invention of claim 13 "a composite magnesium oxide particle represented by the formula (1) for use in a laxative treatment" is "a composite magnesium oxide particle represented by the formula (1)".

As disclosed in Documents 1-3 shown below, the composite particle is already known at the time of filing the present application. Therefore, the common matter cannot be regarded as having any special technical feature.

Consequently, there is no technical relationship involving one or more of the same or corresponding special technical features among those inventions, and those inventions cannot be considered to be so linked as to form a single general inventive concept.

Document 1: JP 06-065011 A (KAISUI KAGAKU KENKYUJO KK) 08 March, 1994
         (08.03.94)
Document 2: JP 08-048606 A (KAISUI KAGAKU KENKYUJO KK) 20 February, 1996
         (20.02.96)
Document 3: JP 11-180808 A (KYOWA CHEM IND CO LTD) 06 July, 1999 (06.07.99)

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9040561 A
- JP 2001048792 A

- JP 2003146889 A